(19) **Europäisches Patentamt**
**European Patent Office**
**Office européen des brevets**

(11) **EP 4 692 352 A1**

(12) **EUROPEAN PATENT APPLICATION**
published in accordance with Art. 153(4) EPC

(43) Date of publication:
**11.02.2026 Bulletin 2026/07**

(21) Application number: **24780546.8**

(22) Date of filing: **27.03.2024**

(51) International Patent Classification (IPC):
**C12N 15/16** (2006.01)   **A01K 67/027** (2024.01)
**C12N 5/075** (2010.01)   **C12N 5/076** (2010.01)

(52) Cooperative Patent Classification (CPC):
**A01K 67/027; C07K 14/575; C12N 5/06**

(86) International application number:
**PCT/JP2024/012460**

(87) International publication number:
**WO 2024/204445 (03.10.2024 Gazette 2024/40)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **27.03.2023 JP 2023050625**

(71) Applicant: **National University Corporation Tokyo University of Marine Science and Technology**
**Minato-ku**
**Tokyo 108-8477 (JP)**

(72) Inventors:
• **YOSHIZAKI Goro**
**Tokyo 108-8477 (JP)**
• **MORITA Tetsuro**
**Tokyo 108-8477 (JP)**
• **MORIYA Natsuko**
**Tokyo 108-8477 (JP)**

(74) Representative: **Zacco Sweden AB**
**P.O. Box 5581**
**Löjtnantsgatan 21**
**114 85 Stockholm (SE)**

Remarks:
The complete document including Reference Table(s) and the Sequence Listing(s) can be downloaded from the EPO website

(54) **METHOD FOR PRODUCING FISH, METHOD FOR PRODUCING FISH AND FISH GAMETES**

(57)  A method for producing fish includes an fsh introduction step (S2) of introducing DNA containing a constitutive promoter and a gene functionally linked to the promoter and encoding follicle-stimulating hormone, into a fertilized egg of fish. In the fish, the constitutive promoter and the gene functionally linked to the promoter and encoding follicle-stimulating hormone are introduced into a genome. A method for producing gametes of fish includes a step of transplanting a germline stem cell of another species of fish into an abdominal cavity of fry of the fish.

**Description**

TECHNICAL FIELD

The present invention relates to a method for producing fish that matures early, fish produced by the method, and a method for producing gametes of fish.

Priority is claimed on Japanese Patent Application No. 2023-050625, filed March 27, 2023, the content of which is incorporated herein by reference.

BACKGROUND ART

In the related art, in the fishery industry, selective breeding of fish having excellent traits such as growth potential, disease resistance, and taste has been carried out.

In the selective breeding, a step of selecting individuals having excellent traits from the same species of fish, crossing the selected individuals, breeding the obtained individuals until maturity, and selecting individuals having excellent traits is repeatedly performed.

However, in a case of selective breeding, it takes a long time for fish to mature to the stage of ovulation, for example, 4 to 5 years for Chum salmon, which is Salmonidae, and about 4 years for Chinook salmon. Therefore, there is a problem in that it takes a long time to carry out selective breeding of fish.

Meanwhile, Patent Document 1 discloses a surrogate broodstock technique in which germ cells of donor fish are transplanted to closely related species serving as a host to produce eggs or sperm.

According to this technique, in a case where germ cells of donor fish are transplanted to closely related fish that matures in a shorter period of time, mature eggs and sperm of the donor fish can be obtained in a shorter period of time than in a case of breeding the donor fish until maturity, and thus the period of time required for selective breeding can be shortened.

The present inventors have actually succeeded in transplanting germ cells of Chinook salmon, which takes about four years to mature, to the fry of rainbow trout and obtaining eggs of Chinook salmon in about two years.

Citation List

Patent Document

Patent Document 1: Republished Japanese Translation No. 2015-146184 of the PCT International Publication for Patent Applications

SUMMARY OF INVENTION

Technical Problem

However, in the surrogate broodstock technique described in Patent Document 1, it is difficult to shorten the production period of a gamete (eggs or sperm) than the period of time required for maturing the host fish.

The present invention has been made in consideration of above-described circumstances, and an object thereof is to provide a method for producing fish, which enables production of a gamete at an early stage from hatching, fish capable of producing a gamete at an early stage from hatching, and a method for producing gametes of fish, which enables production of a gamete at an early stage.

Solution to Problem

The present inventors have initially focused on follicle-stimulating hormone (hereinafter, also referred to as "FSH") and luteinizing hormone (hereinafter, also referred to as "LH"), which are gonadotropic hormones that promote maturation.

Here, it is known that luteinizing hormone binds not only to a receptor of luteinizing hormone but also to a receptor of follicle-stimulating hormone and activates the receptors (Miwa S, Yan L, Swanson P. Localization of two gonadotropin receptors in the salmon gonad by in vitro ligand autoradiography. Biol Reprod 1994; 50: 629-642).

Therefore, the present inventors prepared an expression vector (hereinafter, referred to as hsc:lh) containing a constitutively expressing promoter and an lh gene encoding luteinizing hormone, and carried out microinjection of a DNA chain encoding a promoter region and luteinizing hormone into a fertilized egg of Salmonidae to prepare a mosaic individual (hereinafter, referred to as "lh parent generation mosaic") in which luteinizing hormone is highly expressed.

Thereafter, the present inventors crossed the lh parent generation mosaic with the wild type, selected an lh heterozygous introduced individual (hereinafter, referred to as "lh hetero"), and further crossed the lh hetero with the wild type to carry out the phenotype analysis of the F2 generation of the lh heterozygous individual.

As a result, ejaculation was confirmed at 5.5 months of age in the male individuals of some lh hetero F2, but the ejaculate volume was extremely small. In addition, in the lh hetero F2 females, the ovaries did not develop even at 12 months of age.

Therefore, the present inventors have considered that the amount of collected sperm can be increased and the development of the ovary can be promoted by overexpressing follicle-stimulating hormone that promotes the proliferation and differentiation of spermatogonia and the growth of eggs. Thereafter, the present inventors prepared an expression vector (hereinafter, referred to as hsc:fsh) containing a constitutively expressing promoter and an fsh gene encoding follicle-stimulating hormone, and carried out microinjection of DNA containing the promoter region and the fsh gene into a fertilized egg of Salmonidae to prepare a mosaic individual (hereinafter, referred to as "fsh parent generation mosaic") in which follicle-stimulating hormone is highly expressed and an fsh heterozygous introduced individual (hereinafter, referred to as "fsh hetero") obtained by crossing the fsh parent generation mosaic with a wild type.

As a result, in the fsh parent generation mosaic females, significantly developed ovaries were confirmed at 12 months of age, and ovulation was confirmed at 14.5 months of age. In addition, in the fsh hetero males, an ejaculate volume of 100 μL or greater is confirmed at 6 months of age.

As described above, the present inventors found that the introduction of a constitutive promoter and a DNA chain encoding FSH into a fertilized egg makes it possible to mature both males and females remarkably earlier than in the related art, and thus completed the present invention.

One aspect of the present invention includes the following aspects.

[1] A method for producing fish, including: a step of introducing DNA containing a constitutive promoter and a gene functionally linked to the promoter and encoding follicle-stimulating hormone, into a fertilized egg of fish.

[2] The method for producing fish according to [1], in which the gene has a base sequence encoding an FSHβ chain and a base sequence encoding a GTHα chain, the base sequence encoding the FSHβ chain encodes a protein having an amino acid sequence set forth in SEQ ID NO: 19 or encodes a protein having an amino acid sequence having 30% or more sequence identity with the amino acid sequence set forth in SEQ ID NO: 19, and the base sequence encoding the GTHα chain encodes a protein having an amino acid sequence set forth in SEQ ID NO: 20 or encodes a protein having an amino acid sequence having 60% or more sequence identity with the amino acid sequence set forth in SEQ ID NO: 20.

[3] The method for producing fish according to [1] or [2], in which the promoter is a promoter that controls transcription of an hsc71 gene of fish.

[4] The method for producing fish according to any one of [1] to [3], in which the DNA contains a 3' untranslated region of the hsc71 gene of fish on a downstream side of the base sequence encoding the GTHα chain.

[5] The method for producing fish according to any one of [1] to [4], in which the fish is a female.

[6] The method for producing fish according to any one of [1] to [4], in which the fish is a male.

[7] The method for producing fish according to any one of [1] to [6], in which the fertilized egg is a fertilized egg of Salmonidae.

[8] The method for producing fish according to any one of [1] to [7], in which the fertilized egg is a fertilized egg of the genus Oncorhynchus or the genus Salmo.

[9] The method for producing fish according to [8], in which the fertilized egg is a fertilized egg of rainbow trout.

[10] The method for producing fish according to [7], in which the fertilized egg is a fertilized egg of Salmo salar.

[11] The method for producing fish according to any one of [1] to [10], in which the DNA is linear DNA.

[12] Fish, in which a constitutive promoter and a gene functionally linked to the promoter and encoding follicle-stimulating hormone are introduced into a genome.

[13] The fish according to [12], in which the gene has a base sequence encoding an FSHβ chain and a base sequence encoding a GTHα chain, the base sequence encoding the FSHβ chain encodes a protein having an amino acid sequence set forth in SEQ ID NO: 19 or encodes a protein having an amino acid sequence having 30% or more sequence identity with the amino acid sequence set forth in SEQ ID NO: 19, and the base sequence encoding the GTHα chain encodes a protein having an amino acid sequence set forth in SEQ ID NO: 20 or encodes a protein having an amino acid sequence having 60% or more sequence identity with the amino acid sequence set forth in SEQ ID NO: 20.

[14] The fish according to [12] or [13], in which the promoter is a promoter that controls transcription of an hsc71 gene of fish.

[15] The method for producing fish according to [14], in which a 3' untranslated region of the hsc71 gene of fish is disposed on a downstream side of the base sequence encoding the GTHα chain.

[16] The fish according to any one of [12] to [15], in which the fish is a female.

[17] The fish according to any one of [12] to [15], in which the fish is a male.

[18] The fish according to any one of [12] to [15], in which the fish is Salmonidae.

[19] The fish according to [18], in which the fish is the genus Oncorhynchus or the genus Salmo.

[20] The fish according to [19], in which the fish is rainbow trout.

[21] The fish according to [19], in which the fish is Salmo salar.

[22] A method for producing gametes of fish, including: a step of transplanting a germline stem cell of another species of fish into an abdominal cavity of fry of the fish according to any one of [12] to [21].

Advantageous Effects of Invention

According to the present invention, it is possible to provide a method for producing fish, which enables production of a gamete at an early stage from hatching, fish capable of producing a gamete at an early stage from hatching, and a method for producing gametes of fish, which enables production of a gamete at an early stage.

BRIEF DESCRIPTION OF DRAWINGS

[FIG. 1] A flow showing a method for producing fish according to a preferred embodiment of the present invention.

[FIG. 2] An overall schematic view showing a vector in examples.

[FIG. 3] A view showing a partial base sequence of a vector in examples.

[FIG. 4] A schematic view showing a state of cloning up to 12 bp including a start codon of a promoter region and a coding region of an hsc71 gene.

[FIG. 5] A schematic view showing a state of preparing a vector into which a 3' UTR of an lh gene of rainbow trout and a 3' UTR of an hsc71 gene of rainbow trout are inserted.

[FIG. 6] A schematic view showing a state of constructing hsc:lh in which a hsc71 promoter, an lh gene, eCTP, and a 3' UTR are inserted.

[FIG. 7] A schematic view showing a state in which a portion other than an FSHβ chain coding sequence in a hsc:fsh vector is amplified using hsc:lh as a template.

[FIG. 8] A schematic view showing a state in which an FSHβ chain coding sequence of rainbow trout and a sequence located in the vicinity thereof are amplified.

[FIG. 9] A schematic view showing a state of constructing hsc:fsh into which a hsc71 promoter, an fsh gene, eCTP, and a 3' UTR are inserted.

[FIG. 10A] An image showing a testis of a wild type male individual at 6 months of age.

[FIG. 10B] An image showing a testis of an fsh parent generation mosaic individual at 5.5 months of age.

[FIG. 11] An image showing a state in which an fsh parent generation mosaic male individual at 13 months of age is viewed from a side.

[FIG. 12] An image showing a state in which an fsh parent generation mosaic male individual at 13 months of age is viewed from below.

[FIG. 13] An HE-stained image of an ovary of a wild type individual at 12 months of age.

[FIG. 14] An HE-stained image of an ovary of a exogenous lh gene-introduced individual at 12 months of age.

[FIG. 15] An image showing a state in which an abdomen of a wild type individual at 12 months of age is opened.

[FIG. 16] An image showing a state in which an abdomen of an fsh parent generation mosaic individual at 12 months of age is opened.

[FIG. 17] A stereomicroscope photograph of eggs collected from an fsh parent generation mosaic individual at 12 months of age.

[FIG. 18] A stereomicroscope photograph of eggs collected from an fsh parent generation mosaic individual at 14.5 months of age and a partially enlarged view thereof.

[FIG. 19A] A fluorescence image of gonads of a 2N fsh heterozygous individual into which spermatogonia of vasa-gfp gene-introduced rainbow trout were transplanted.

[FIG. 19B] A fluorescence image of gonads of a 3N exogenous fsh-introduced infertile individual into which spermatogonia of vasa-gfp gene-introduced rainbow trout were transplanted.

[FIG. 19C] A fluorescence image of gonads of a 2N wild type individual into which spermatogonia of vasa-gfp gene-introduced rainbow trout were transplanted.

[FIG. 19D] A fluorescence image of gonads of a 3N infertile individual having a genome derived from a wild type individual into which spermatogonia of vasa-gfp gene-introduced rainbow trout were transplanted.

[FIG. 20] An image showing the results of PCR amplification of a gfp gene using a genome of sperm collected from a host of fsh hetero 3N at 5 months of age and 6 months of age as a template and electrophoresis.

[FIG. 21] A microscopic image showing fertilized eggs produced by fertilizing eggs collected from a wild type female individual with sperm collected from an fsh hetero 3N individual which is a host, the sperm being derived from spermatogonia collected from individuals of rainbow trout which are dominant albino gene heterozygotes.

[FIG. 22A] A fluorescence image of gonads of an fsh hetero 3N individual.

[FIG. 22B] A fluorescence image of gonads of a wild type 3N individual.

[FIG. 23] An electrophoresis image obtained by performing species discrimination analysis using a restriction fragment length polymorphism (RFLP) method with a genome of sperm collected from an fsh hetero 3N individual of rainbow trout at 8 months of age into which spermatogonia derived from Chinook salmon were injected.

DESCRIPTION OF EMBODIMENTS

Hereinafter, preferred embodiments of the present invention will be described in detail with reference to the accompanying drawings as necessary.

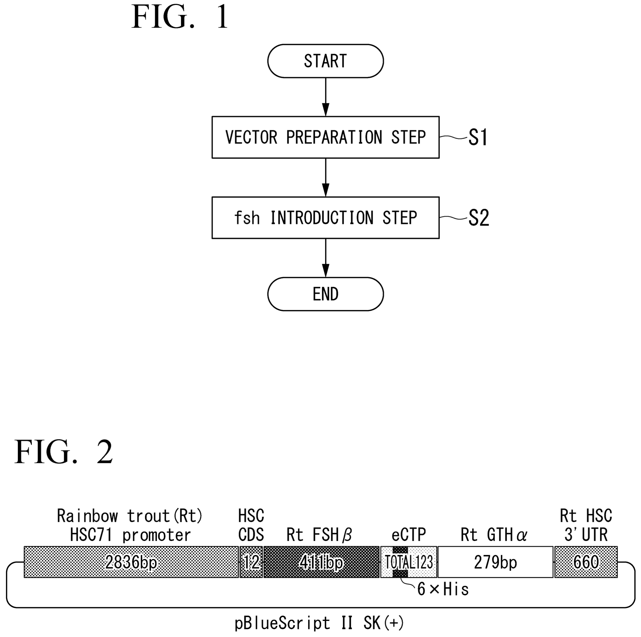

FIG. 1 is a flow showing a method for producing fish according to a preferred embodiment of the present invention.

The method for producing fish according to the present embodiment includes a step of preparing a vector containing an fsh gene functionally linked to a constitutive promoter (vector preparation step S1), and a step of introducing a part or the whole of a sequence of the vector, which contains the constitutive promoter and the fsh gene, into a fertilized egg of fish (fsh introduction step S2).

(Fish)

The species of fish produced by the production method of the present invention is not limited. Therefore, for example, horse mackerel fish, mackerel fish, eel, and the like may be produced by the present production method, but salmonid fish is particularly preferable. Among these, any fish of the genus Oncorhynchus (Pacific salmon), the genus Salmo, the genus Salvelinus, the genus Parahucho, the genus Thymallus, the genus Coregonus, the genus Stenodus, the genus Brachymystax, or the genus Prosopium may be used, but fish of the genus Oncorhynchus or the genus Salmo is preferable. In addition, the fish produced by the present production method may be a female or a male.

Examples of the fish of the genus Oncorhynchus, which is produced by the production method of the present invention, include salmon (Chum salmon), Sockeye salmon, coho salmon, masu salmon, pink salmon, Chinook salmon, rainbow trout, Salmo salar such as so-called Norwegian salmon, and brown trout, but the present invention is not limited thereto.

(Vector preparation step S1)

The vector prepared in the vector preparation step S1 is not particularly limited as long as the vector contains a constitutive promoter in fish (in other words, a constitutively expressing promoter, a promoter having constitutive activity) and an fsh gene encoding follicle-stimulating hormone FSH transferably linked (in other words, operable or functional) to the promoter.

The kind of the constitutive promoter in fish is not particularly limited, and examples thereof include a promoter that controls transcription of each gene encoding a heat shock cognate protein (HSC), an RSVLTR, a mammalian-derived SV40 promoter, an RSV promoter, a miw promoter, a UCP promoter, a cytomegalovirus promoter, a medaka β-actin promoter, and a Mylz2 promoter.

Among these, it is preferable to use a promoter of a heat shock cognate protein, which is a heat shock protein that can be constitutively expressed throughout the body containing gonads in which an FSH receptor and an LH receptor are expressed. Examples of the promoter of the heat shock cognate protein include promoters that control transcription of genes such as hsc70, hsc71, and hsc73, but the present invention is not limited thereto.

The fsh gene encoding follicle-stimulating hormone (FSH) is preferably an fsh gene of fish and more preferably an fsh gene of Salmonidae.

The fsh gene is widely conserved among various species, and has a base sequence encoding an FSHβ chain (hereinafter, referred to as "FSHβ chain coding sequence") and a base sequence encoding a GTHα chain (hereinafter, referred to as "GTHα chain coding sequence").

As the FSHβ chain coding sequence, a base sequence encoding an FSHβ chain of rainbow trout or an FSHβ chain having an amino acid sequence similar to that of the FSHβ chain of the rainbow trout is particularly useful. The FSHβ chain coding sequence of rainbow trout is a base sequence set forth in SEQ ID NO: 1, and encodes an amino acid sequence set forth in SEQ ID NO: 19. The FSHβ chain coding sequence that encodes a protein having an amino acid sequence set forth in SEQ ID NO: 19 or a protein having an amino acid sequence having 30% or more (preferably 40% or more and more preferably 90% or more) sequence identity with the amino acid sequence set forth in SEQ ID NO: 19 can be suitably used. The protein having an amino acid sequence having 30% or more (preferably 40% or more and more preferably 90% or more) sequence identity with the amino acid sequence set forth in SEQ ID NO: 19 is a protein having an activity of promoting gametogenesis as an FSHβ chain.

Further, for example, an amino acid sequence of a β-subunit of a human HCG hormone that exhibits a strong maturation-inducing effect on horse mackerel or chub mackerel has about 30% sequence identity with an amino acid sequence of a β-subunit of luteinizing hormone of horse mackerel or chub mackerel. Therefore, the numerical range of 30% or more is a numerical range that is not extremely low for functioning as FSHβ.

In the present specification, the sequence identity of the target amino acid sequence with respect to the reference amino acid sequence (for example, the amino acid sequence set forth in SEQ ID NO: 19 or 20) can be determined as follows.

First, the reference amino acid sequence and the target amino acid sequence are aligned. Each amino acid sequence has a gap such that the sequence identity is maximized. Next, the number of amino acids that match between the reference amino acid sequence and the target amino acid sequence can be calculated, and the sequence identity can be calculated according to Equation (1).

Sequence identity (%) = number of amino acids matched/total number of amino acids in target amino acid sequence $\times$ 100     (1)

As the GTHα chain coding sequence, a base sequence encoding a GTHα chain of rainbow trout or a GTHα chain having an amino acid sequence similar to that of the GTHα chain of rainbow trout is preferable.

The GTHα chain coding sequence of rainbow trout is a base sequence set forth in SEQ ID NO: 2, and encodes an amino acid sequence set forth in SEQ ID NO: 20. The GTHα chain coding sequence that encodes a protein having the amino acid sequence set forth in SEQ ID NO: 20 or encodes a protein having an amino acid sequence having 60% or more (preferably 70% or more and more preferably 90% or more) sequence identity with the amino acid sequence set forth in SEQ ID NO: 20 can be suitably used. The protein having an amino acid sequence having 60% or more (preferably 70% or more and more preferably 90% or more) sequence identity with the amino acid sequence set forth in SEQ ID NO: 20 is a protein having an activity of promoting gametogenesis as a GTHα chain.

By functionally linking (in other words, transferably linking) such an fsh gene to any of the above-described promoters, follicle-stimulating hormone can be constitutively expressed in the body of fish, and the production of gametes can be promoted.

In addition, in the fsh gene inserted into the vector, another sequence may be inserted between the FSHβ chain coding sequence and the GTHα chain coding sequence.

For example, by inserting an eCTP sequence (see SEQ ID NO: 3) encoding a C-terminal peptide derived from equine luteinizing hormone/chorionic gonadotropin between an FSHβ chain coding sequence and a GTHα chain coding sequence, it is possible to impart more sugar than the follicle-stimulating hormone of a wild-type individual, and the resulting FSH becomes less likely to be degraded in vivo.

In addition, only the protein derived from a exogenous fsh gene can be purified without affecting the activity of the exogenous fsh gene by inserting six His tags (base sequences encoding six consecutive histidines) into the eCTP sequence as in the base sequence set forth in SEQ ID NO: 3.

In a case where hsc71 of fish is used as the promoter, strong expression of the fsh gene can be realized by inserting a 3' UTR on a downstream side of the fsh gene. In addition, translation can be smoothly promoted even in a case where a promoter region of a gene other than the fsh gene is used as the promoter region by allowing the start codon of the hsc71 gene to be contained between the promoter region of the hsc71 gene and the fsh gene.

In addition, the FSHβ chain coding sequence and the GTHα chain coding sequence are preferably inserted into one vector, but may be inserted into separate vectors. In this case, it is necessary to introduce both vectors having any of the sequences into the fertilized egg.

The kind of the expression vector into which the fsh gene and the promoter controlling the transcription of the fsh gene are inserted is not particularly limited, and examples thereof include a bacterial plasmid such as pBlueScript II SK+ (manufactured by Stratagene), pGEM-T Easy Vector (manufactured by Promega Corporation), and pRcRSV (Invitrogen).

The expression vector may include a selective marker such as an antibiotic resistance gene for selection or a bacterial replication origin sequence.

The fsh gene can be linked to these vectors by various methods such as an In-Fusion reaction.

Further, it is not always necessary to prepare an expression vector containing the fsh gene and the constitutive promoter from scratch, and such an expression vector may be obtained and used in the fsh introduction step S2.

(fsh introduction step S2)

In the fsh introduction step S2, a part or the entirety of the vector prepared in the vector preparation step S1, which contains the fsh gene and the promoter, is introduced into the fertilized egg of the fish, thereby introducing a sequence containing the fsh gene and the promoter into the genome of the fertilized egg.

In a case where only a part of the vector is introduced into the fertilized egg and the circular plasmid vector of the present embodiment is used as the expression vector, the vector prepared in the vector preparation step S1 needs to be cut into a part containing the fsh gene and the promoter and a part of the other vector with a restriction enzyme or the like, prior to introduction of the vector to the fertilized egg.

As described above, by cutting out a part of the circular vector, forming linear DNA containing a constitutive promoter and an fsh gene, and introducing the linear DNA into a fertilized egg, the efficiency of introducing the constitutive promoter and the fsh gene into the genome can be further increased as compared with a case of introducing the circular DNA into the fertilized egg.

Although microinjection is desirable for the introduction of the entire vector or a part thereof into the fertilized egg, the method for introducing the vector into the fertilized egg is not limited thereto.

The fish produced as described above is not limited to an individual into which the above-described vector is directly introduced, that is, an individual of the fsh parent generation mosaic containing the introduced exogenous fsh gene, and the exogenous fsh gene is constitutively expressed even in the fsh heterozygous individual and the fsh homozygous individual obtained by crossing the fsh parent generation mosaic individual with a wild type individual, crossing the fsh parent generation mosaic individuals with each other, crossing the fsh parent generation mosaic individual with an fsh heterozygous individual, crossing the fsh heterozygous individuals with each other, and the like.

As a result, it is expected that these individuals mature significantly faster than individuals of the same species of fish in the related art, and produce gametes early from hatching.

As described in detail in examples below, the development of the ovary is not observed even at 12 months of age in the lh hetero female individual in which LH is overexpressed, but the ovary is largely developed at 12 months of age and ovulation can be confirmed at 14.5 months of age in the fsh parent generation mosaic individual. In addition, in the fsH hetero male individual, it can be confirmed that the ejaculate volume is greater than 100 μL at 6 months of age.

Further, the method for producing fish described above can also be used as a method for producing fish which enables production of gametes at an early stage after hatching.

[Other embodiments]

In one embodiment, the present invention provides fish in which a constitutive promoter and an fsh gene that is functionally linked to the promoter and encodes follicle-stimulating hormone are introduced into a genome.

The fish according to the present embodiment is an individual of fish produced by the above-described various production methods and, among the fry thereof, an individual in which a exogenous constitutive promoter and the fsh gene are introduced into a genome, and the fish according to the present embodiment matures in a remarkably short period of time and enters a state of producing gametes, as compared with the wild type individual of the same species in the related art. Further, the fish according to the embodiment may be a female or a male.

Various promoters described in detail in the above-described production method, for example, the promoter that controls transcription of the hsc71 gene of Salmonidae can be suitably used as the promoter according to the present embodiment, but the promoter is not particularly limited as long as it is a promoter capable of constitutively expressing the fsh gene in fish.

In a case where a promoter that controls transcription of the hsc71 gene is used, it is preferable that a 3' untranslated region is disposed on a downstream side of the GTHα chain coding sequence of the fsh gene.

As the fsh gene according to the present embodiment, the fsh gene described in detail in the above-described production method, that is, the fsh gene of fish can be suitably used, but as described above, the fsh gene of Salmonidae is more preferable.

The fsh gene has an FSHβ chain coding sequence and a GTHα chain coding sequence. Among these, an fsh gene having an FSHβ chain coding sequence that encodes a protein having the amino acid sequence set forth in SEQ ID NO: 19 or a protein having an amino acid sequence having 30% or more (preferably 40% or more and more preferably 90% or more) sequence identity with the amino acid sequence set forth in SEQ ID NO: 19 can be suitably used as the FSHβ chain coding sequence, and an fsh gene having a GTHα chain coding sequence that encodes a protein having the amino acid sequence set forth in SEQ ID NO: 20 or a protein having an amino acid sequence having 60% or more (preferably 70% or more and more preferably 90% or more) sequence identity with the amino acid sequence set forth in SEQ ID NO: 20 can be suitably used as the GTHα chain coding sequence. The protein having an amino acid sequence having 30% or more (preferably 40% or more and more preferably 90% or more) sequence identity with the amino acid sequence set forth in SEQ ID NO: 19 is a protein having an activity of promoting gametogenesis, as the FSHβ chain. The protein having an amino acid sequence having 60% or more (preferably 70% or more and more preferably 90% or more) sequence identity with the amino acid sequence set forth in SEQ ID NO: 20 is a protein having an activity of promoting gametogenesis as a GTHα chain.

Such a sequence is effective for all fish, but is particularly effective for Salmonidae, particularly fish of the genus Oncorhynchus such as rainbow trout and fish of the genus Salmo closely related to fish of the genus Oncorhynchus (for example, Pacific salmon), and can produce gametes in a short period of time from hatching.

In another embodiment, the present invention provides a method for producing gametes of fish, including a step of transplanting a germline stem cell of another species of fish into an abdominal cavity of fry of the fish into which a constitutive promoter and a gene functionally linked to the promoter and encoding follicle-stimulating hormone are introduced into a genome.

The expression "fish into which a gene functionally linked to the promoter and encoding follicle-stimulating hormone is introduced into a genome" according to the embodiment is the same as the fish according to the above-described "other embodiments". Therefore, the fsh gene described in detail in the above-described production method can be used as the gene encoding follicle-stimulating hormone. That is, the fsh gene may have an FSHβ chain coding sequence and a GTHα chain coding sequence.

As the FSHβ chain coding sequence, a sequence encoding a protein having the amino acid sequence set forth in SEQ ID NO: 19, or a sequence encoding a protein having an amino acid sequence having 30% or more (preferably 40% or more and more preferably 90% or more) sequence identity with the amino acid sequence set forth in SEQ ID NO: 19 can be suitably used.

The protein having an amino acid sequence having 30% or more (preferably 40% or more and more preferably 90% or more) sequence identity with the amino acid sequence set forth in SEQ ID NO: 19 is a protein having an activity of promoting gametogenesis, as the FSHβ chain.

As the GTHα chain coding sequence, a GTHα chain coding sequence that encodes a protein having the amino acid sequence set forth in SEQ ID NO: 20 or a protein having an amino acid sequence having 60% or more (preferably 70% or more and more preferably 90% or more) sequence identity with the amino acid sequence set forth in SEQ ID NO: 20 can be suitably used.

The protein having an amino acid sequence having 60% or more (preferably 70% or more and more preferably 90% or more) sequence identity with the amino acid sequence set forth in SEQ ID NO: 20 is a protein having an activity of promoting gametogenesis as a GTHα chain.

For example, by transplanting germline stem cells of Salmo salar, Chinook salmon, or the like into the abdominal cavity of the fry of fsh rainbow trout into which the exogenous fsH gene and the constitutive promoter have been introduced, it is possible to produce gametes of Salmo salar, Chinook salmon, or the like in a shorter period of time than in the related art.

Further, the concept of "introduced fish" here does not refer only to an individual in which a constitutive promoter and an fsh gene are directly introduced at the stage of a fertilized egg, but also refers to all individuals after F1, which are the fry of the individual and have a exogenous constitutive promoter and an fsh gene.

As the fry in the present embodiment, the fry of the fish produced by the above-described production method can be used.

Various promoters described in detail in the above-described production method, for example, a promoter that controls transcription of an hsc71 gene of fish can be suitably used as the promoter according to the present embodiment, but the promoter is not particularly limited as long as the promoter can constitutively express an fsh gene in fish.

The production method according to the present embodiment is also useful as a method for producing gametes of fish capable of producing gametes at an early stage.

It is preferable that the introduction of the fsh gene and the constitutive promoter region into the abdominal cavity of the fry is performed from 3 days before hatching to 3 days after hatching of the fry, but the present invention is not limited to this period of time.

According to this embodiment, the fry as a host matures early, but in a case where germline stem cells derived from a wild type individual are used for the germline stem cells to be introduced into the abdominal cavity of the fry, the gametes are wild type gametes that do not contain exogenous genes. Therefore, a wild type individual can also be produced by using the gametes, and for example, a wild type individual which matures slowly can also be selected in a breeding farm.

In addition, according to this embodiment, as described above, since a wild type gamete (gamete containing no exogenous gene) can also be produced, non-genetically modified fish can be produced while the production of the gamete speeds up. Therefore, it is possible to provide fish that even customers who feel resistance to genetically modified fish do not feel resistance to.

The present invention is not limited to the embodiments described above, and various modifications can be made within the scope of the invention described in the claims, and it goes without saying that those modifications are also included in the scope of the present invention.

Examples

Hereinafter, the present invention will be described based on the examples, but the present invention is not limited to the following examples.

(Vector preparation step S1)

A vector according to the present example has in the following order, a promoter region (SEQ ID NO: 21) of an hsc71 gene derived from rainbow trout, a CDS (12 bp, SEQ ID NO: 22) having a start codon of the hsc71 gene consecutive to the promoter region, an FSHβ chain coding sequence (SEQ ID NO: 1) having a signal sequence required for secretion of a protein, an eCTP sequence (SEQ ID NO: 3) encoding a C-terminal peptide derived from equine luteinizing hormone/chorionic gonadotropin, a GTHα coding sequence (SEQ ID NO: 2) having no signal sequence, and a 3' untranslated region (3' UTR, SEQ ID NO: 23) of the hsc71 gene derived from rainbow trout, which is provided on a downstream side of the fsh gene (see FIG. 1).

In addition, SEQ ID NO: 24 shows a base sequence in a state where these sequences are connected.

In addition to the sequence of the 3' UTR of rainbow trout, which is described in S85730.1 of GenBank of NCBI, the following documents are also referred to.

Zafarullah, M., J. Wisniewski, N. W. Shworak, S. Schieman, S. Misra, and L. Gedamu. 1992. "Molecular Cloning and Characterization of a Constitutively Expressed Heat-Shock-Cognate hsc71 Gene from Rainbow Trout." European Journal of Biochemistry/FEBS 204 (2): 893-900.

As described above, by allowing a short sequence having a start codon of the hsc71 gene to be contained on an immediately downstream side of the promoter region of the hsc71 gene, translation can be smoothly promoted even in a case where the coding region is derived from a gene different from the promoter region.

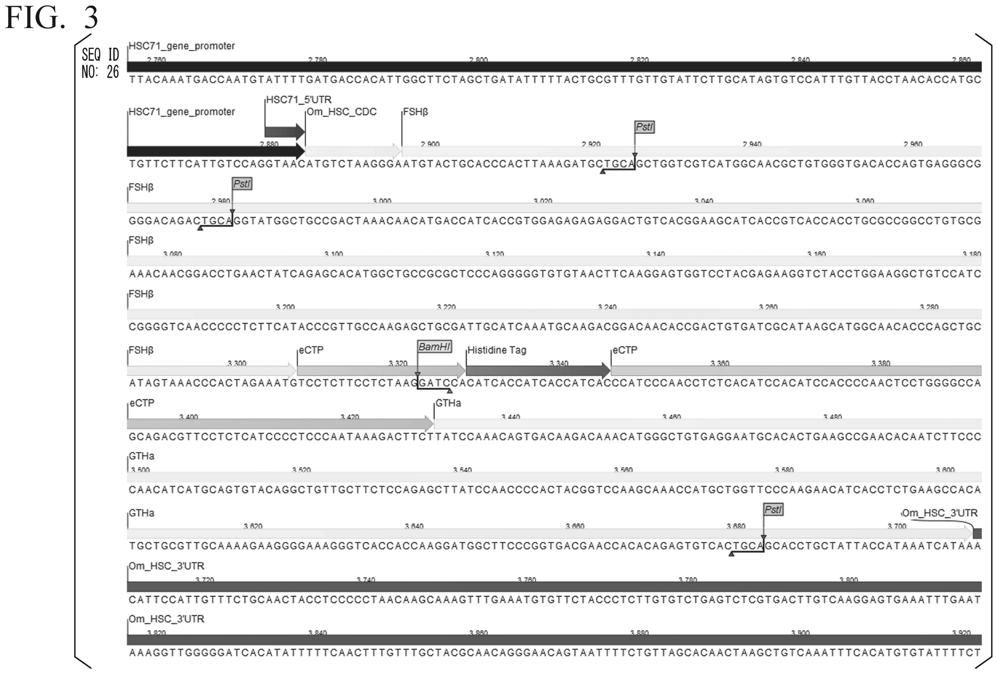

Further, FIG. 2 shows an overall schematic view of the above-described vector, and FIG. 3 shows a base sequence from a downstream portion in the promoter region of the hsc71 gene to an upstream portion in the 3' UTR.

In the present example, based on an hsc:lh vector having the promoter region of the exogenous lh gene and hsc71, an hsc:fsh was prepared by substituting a sequence encoding the LHβ chain with an FSHβ chain coding sequence, and a part of the hsc:fsh containing the exogenous fsh gene was introduced into the fertilized egg.

Hereinafter, first, a method of preparing an hsc:lh vector will be described in detail.

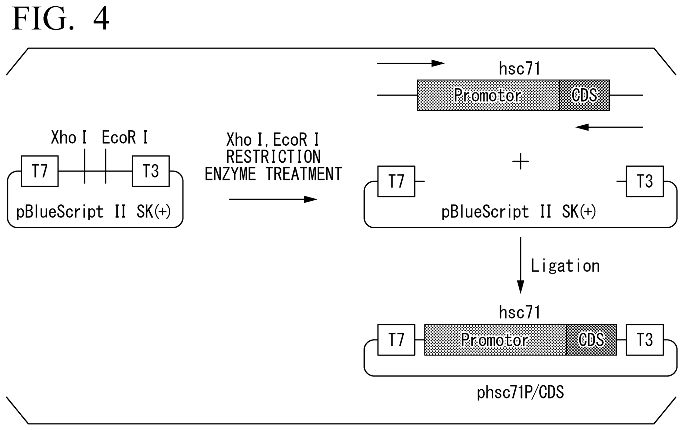

In order to create the above-described vector, first, a promoter region (2836 bp) and 12 bp having a start codon of a coding region of the hsc71 gene from the genome of the rainbow trout were cloned into pBlueScript II SK+ (manufactured by Stratagene) (see FIG. 4).

Specifically, first, pBlueScript II SK+ was cut with restriction enzymes XhoI (manufactured by Takara Bio Inc.) and EcoRI (manufactured by Takara Bio Inc.) and ring-opened (see FIG. 4).

Next, the DNA sample after the operation was subjected to agarose gel electrophoresis, a band having an expected fragment length (2888 bp) was cut out from the agarose gel, and the ring-opened linear pBlueScript II SK+ was purified using a FastGene Gel/PCR Extraction Kit (manufactured by Fast Gene Co., Ltd).

Thereafter, a PCR reaction was carried out in a 20 μL reaction system containing PrimeSTAR Max DNA Polymerase (manufactured by Takara Bio Inc.) using the primer shown in SEQ ID NO: 4 and SEQ ID NO: 5, with the rainbow trout genome as a template, in order to increase the sequence of 12 bp from the start codon of the coding region consecutive to the promoter region (2836 bp) of the hsc71 gene of the rainbow trout inserted.

As the reaction conditions, after the thermal denaturation at 98°C for 10 seconds at the start of the reaction, a series of reactions (98°C for 10 seconds, 55°C for 5 seconds, and 72°C for 15 seconds) were repeated 35 times. After completion of the PCR reaction, the sample was subjected to electrophoresis and cut out from the agarose gel for purification in the same manner as described above.

Next, the purified product was subjected to ligation using an In-Fusion (registered trademark) HD Cloning Kit (manufactured by Takara Bio Inc.) in order to insert the product into the ring-opened pBlueScript II SK+, thereby constructing phsc71P/CDS (FIG. 4).

Thereafter, the 3' UTR of the lh gene of the rainbow trout and the 3' UTR of the hsc71 gene of the rainbow trout were subjected to multi-cloning for being inserted into pBlueScript II SK+.

Specifically, first, in order to amplify the lh gene of the rainbow trout which is an insert, a DNA chain (see SEQ ID NO: 25) in which an FSHβ chain coding sequence (SEQ ID NO: 1), an eCTP sequence (SEQ ID NO: 3), and a GTHα coding sequence (SEQ ID NO: 2) were connected in this order was used as a template, and the primers shown in SEQ ID NO: 6 and SEQ ID NO: 7 were used. In addition, in order to amplify the 3' UTR (660 bp) of the hsc71 gene of the rainbow trout, the genome of the rainbow trout was used as a template, the primers shown in SEQ ID NO: 8 and SEQ ID NO: 9 were used and as a result, the PCR reaction was carried out in a 20 μL reaction system containing PrimeSTAR Max DNA Polymerase (manufactured by Takara Bio Inc.). Further, the PCR reaction was carried out under the same conditions as those for phsc71P/CDS. In addition, a part of the primer of SEQ ID NO: 7 is a sequence that is bound to the GTHα coding sequence, and a sequence of a part that is not bound to the GTHα coding sequence is the same sequence as a part of the 3' UTR (SEQ ID NO: 23) of the hsc71 gene. In this manner, the amplified lh gene of the rainbow trout and the 3' UTR can be easily linked to each other without using an enzyme or the like.

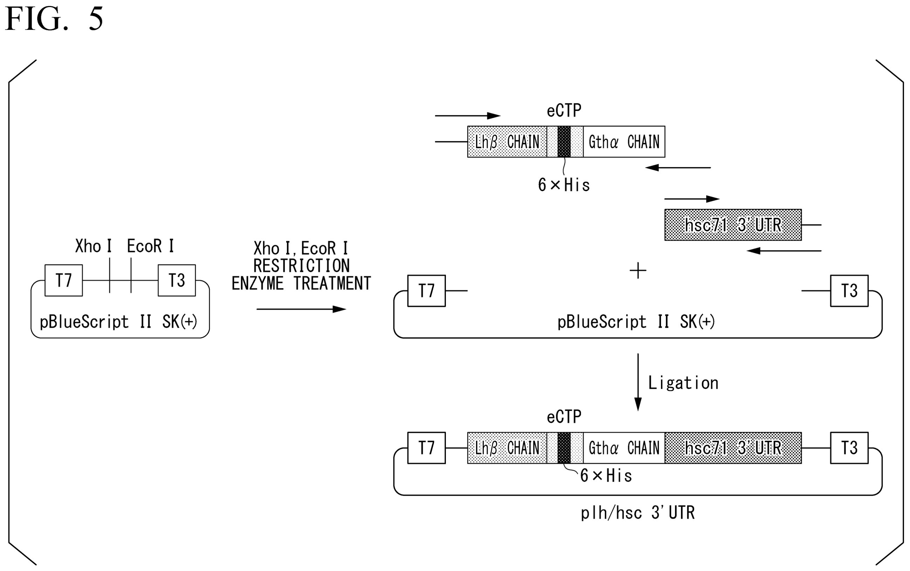

After the PCR reaction, the cut-out purification was performed, and the plh/hsc3' UTR was prepared by ligating to pBlueScriptII SK+ subjected to a restriction enzyme treatment at the time of creating phsc71P/CDS using an In-Fusion HD Cloning Kit (manufactured by Takara Bio Inc.) (see FIG. 5).

Next, the primers of SEQ ID NO: 10 and SEQ ID NO: 11 were used with phsc71P/CDS as a template in order to amplify a 12 bp sequence (total of 2848 bp) having the promoter region and the start codon of the coding region of the hsc71 gene, and the primers of SEQ ID NO: 9 and SEQ ID NO: 12 were used with plh/hsc3' UTR as a template in order to amplify a sequence in which the 3' UTR of the hsc71 gene was connected to the lh gene of rainbow trout, and a PCR reaction was carried out in a 20 μL reaction system containing PrimeSTAR Max DNA Polymerase. This PCR reaction was carried out under the same conditions as those for phsc71P/CDS.

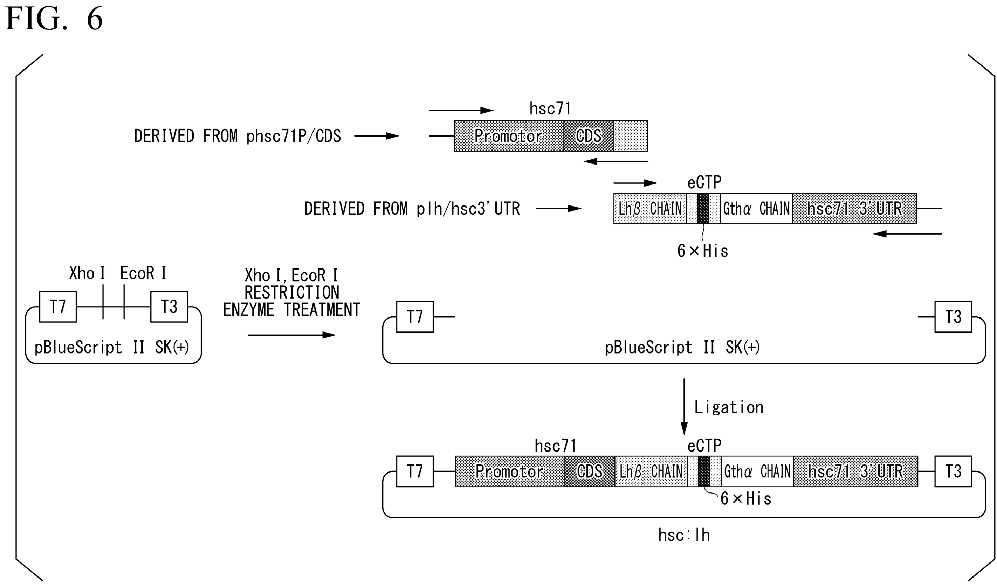

After the PCR reaction, the cut-out purification was carried out, and hsc:lh, which is an lh expression vector was constructed by multi-cloning to pBlueScript II SK+ subjected to a restriction enzyme treatment at the time of creating phsc71P/CDS using the In-Fusion HD Cloning Kit (see FIG. 6).

Based on the hsc:lh vector created as described above, an hsc:fsh vector was prepared as follows.

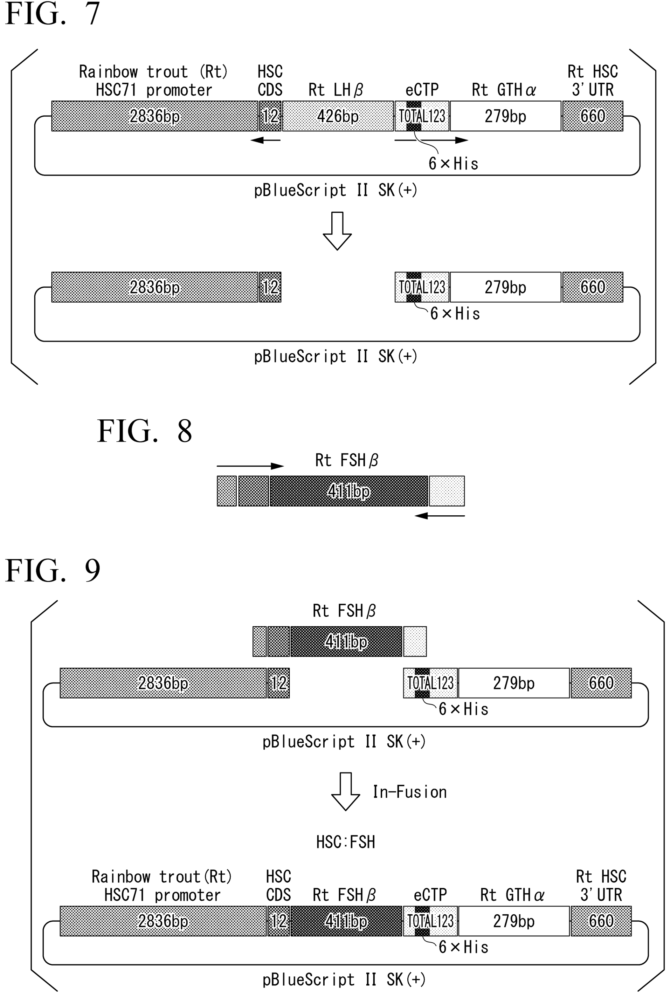

First, the hsc:lh described above was used as a template, and a part other than the FSHβ chain coding sequence was amplified in the hsc:fsh vector using SEQ ID NOs: 13 and 14 (see FIG. 7).

Next, total RNA was extracted from the pituitary gland of an immature wild type individual of rainbow trout at 13 months of age using ISOGEN (manufactured by Nippon Gene Co., Ltd.). Next, 450 μL of a decomposition reaction solution containing 6 U of RQ1 RNase-Free DNase (manufactured by Promega Corporation) was added to 200 ng of the extract, and a DNase treatment was carried out.

Thereafter, phenol extraction was performed, and further, RNA purification was performed by ethanol precipitation.

Next, cDNA was synthesized using 200 ng of the obtained total RNA as a template and a PrimeScript (registered trademark) 1st strand cDNA Synthesis Kit (manufactured by Takara Bio Inc.).

Thereafter, the FSHβ chain coding sequence of rainbow trout and the sequence located in the vicinity thereof were amplified using the obtained cDNA as a template and the primers of SEQ ID NOs: 15 and 16 (see FIG. 8).

Finally, the obtained FSHβ chain coding sequence and a part of the hsc:fsh vector other than the FSHβ chain coding sequence were connected using the In-Fusion HD Cloning Kit, thereby preparing hsc:fsh (see FIG. 9).

This expression vector is capable of expressing each subunit protein as a fusion protein in a single-stranded state in order to express the FSHβ chain and the GTHα chain in association with each other in vivo.

(fsh introduction step S2)

In the present example, the above-described hsc:fsh vector is subjected to a cutting treatment with restriction enzymes XhoI and EcoRI, and linear DNA containing the promoter region of hsc71, 12 bp of the CDS of hsc71, the FSHβ chain coding sequence, eCTP, the GTHα chain coding sequence, and the 3' UTR of hsc71 is cut out and introduced into fertilized eggs.

In this manner, the efficiency of introducing into the genome can be increased by introducing the DNA containing the fsh gene and the constitutive promoter region into the fertilized egg in a linear DNA state.

The DNA that had been subjected to a cutting treatment with restriction enzymes XhoI and EcoRI was cut out from a gel and purified to remove the vector sequence, and diluted to 30 ng/μL in an injection buffer containing 10 mM (mol/l) Tris-HCl (pH 8.0) and 0.1 M EDTA, thereby preparing a DNA solution.

For the creation of fertilized eggs, females and males of rainbow trout at 3 years and 2 years of age, which had been bred at a water temperature of 10.5°C, were used at the Aquatic Science Field Education and Research Center, Oizumi Station, Tokyo University of Marine Science and Technology. In addition, an Oizumi system in which sexual maturity reached at 2 years of age for males and 3 years of age for females was used as the males and females of fish.

Gametes were collected from each of the male and female individuals by a squeezing method, the eggs were washed by an isotonic egg washing method, and the gametes were inseminated by a 1.1% NaHCO$_3$ immersion method, thereby obtaining fertilized eggs.

About 2 μL of the above-described DNA solution was microinjected into each of the one-cell stage fertilized eggs that had been subjected to water absorption in a 1 mM reduced glutathione solution (pH 8.0).

The fertilized eggs after the microinjection were returned to the environmental water and housed in a basket made of a mesh with satisfactory water conduction until hatching.

Thereafter, the hatched juvenile fish were fed 5 to 7 times a day.

At a stage at which these individuals reached 3 months of age, individuals into which the exogenous fsh gene had been introduced were searched for.

Specifically, first, a PCR reaction was performed on genomic DNA extracted from the adipose fin of each individual using introduced fsh gene-specific primers of SEQ ID NO: 17 (Fw) and SEQ ID NO: 18 (Rv) in a 10 μL reaction system containing Takara Ex Taq (manufactured by Takara Bio Inc.). After the thermal denaturation at 94°C for 3 minutes, the reaction was started and repeatedly carried out at a series of cycles of 94°C for 30 seconds, 58°C for 30 seconds, and 72°C for 30 seconds 40 times.

After completion of the PCR reaction, 1 μL of each reaction solution was subjected to electrophoresis using a 2% agarose gel as a carrier, and the presence or absence of amplification of the fsh gene was confirmed. As a result, the fsh parent generation mosaic individual in which the band of the introduced fsh gene was confirmed was bred in a 200 L water tank.

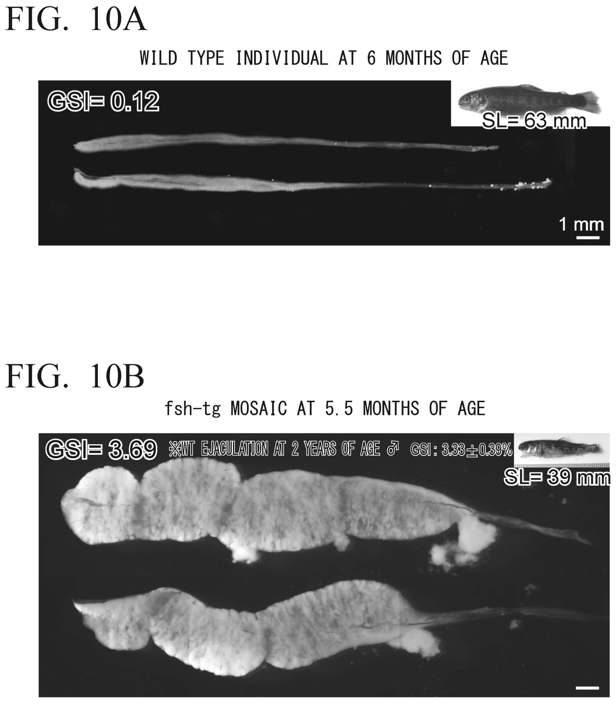

Thereafter, it was confirmed that the testis was highly developed in the fsh parent generation mosaic male individual (see FIG. 10B) at 5.5 months of age as compared with the wild type male individual (see FIG. 10A) at 6 months of age. In a case where a Gonadosomatic Index (GSI) indicating the weight of the gonad per body weight was compared, the GSI of the wild type individual at 6 months of age was 0.12, whereas the GSI of the fsh parent generation mosaic individual at 5.5 months of age was 3.69. This value is equal to or greater than that of ejaculating wild-type males at 2 years of age.

The density of sperm collected from the lh hetero F2 generation individual at 6 months of age into which the exogenous lh gene derived from hsc:lh described above had been introduced was $1.5 \times 10^8$ cells/mL, whereas the sperm density of fsh parent generation mosaic individual at 6 months of age was $8.9 \times 10^8$ cells/mL. The sperm of the fsh parent generation mosaic individual at 6 months of age was morphologically normal.

[Table 1]

| Test section | Fertilization rate | Hatching rate (number of hatches) | Number of test eggs | Amount of collected sperm (μL) | Density of sperm (cells/mL) |
|---|---|---|---|---|---|
| lh-tg (1) | 87.5% | 79.1% (189) | 239 | 16 | $1.5 \times 10^8$ |
| lh-tg (2) | 29.2% | 13.3% (33) | 248 | 7 | $8.5 \times 10^6$ |
| Wild type | 100% | 100% (50) | 50 | - | - |

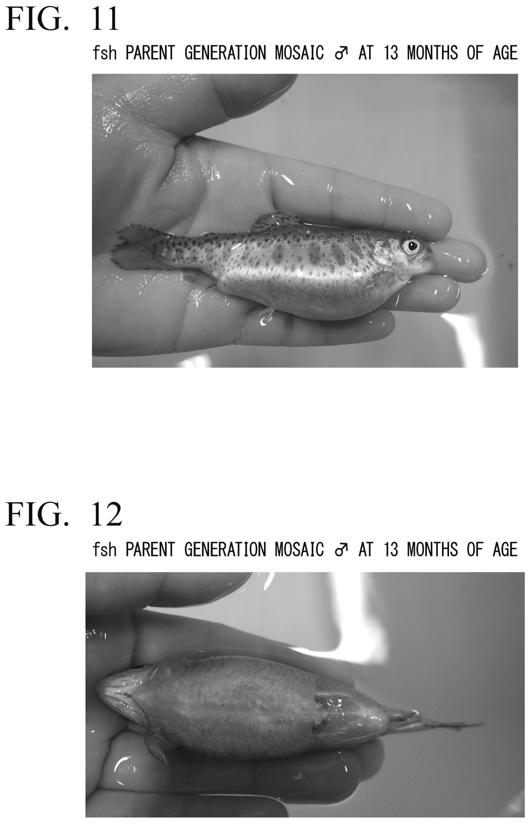

A male individual of the fsh parent generation mosaic at 13 months of age, the abdomen is seen to be greatly stretched and the testis is seen to be greatly developed as shown in FIG. 11 (image in which the individual is observed from the side) and FIG. 12 (image in which the individual is observed from the bottom surface).

In addition, an fsh hetero F1 generation was produced by fertilizing eggs collected from a wild type individual with sperm collected from an fsh parent generation mosaic individual at 6 months of age.

In the fsh hetero F1 generation, ejaculation was confirmed in 14 out of 15 individuals at 6 months of age.

[Table 2]

| Test section | Fertilization rate | Hatching rate (number of hatches) | Number of test eggs | Amount of collected sperm (μL) | Density of sperm (cells/mL) |
|---|---|---|---|---|---|
| fsh-tg (1) | 100% | 99.3% (450) | 450 | 116 | $6.9 \times 10^9$ |
| fsh-tg (2) | 95.8% | 97.3% (403) | 403 | 157 | $5.4 \times 10^9$ |
| Wild type | 100% | 99.0% (102) | 103 | - | - |

Although the density of sperm collected from the fsh hetero F1 generation individual at 6 months of age varies depending on the test section, the densities thereof are $6.9 \times 10^9$ cells/mL and $5.4 \times 10^9$ cells/mL, which were greater than that of the lh hetero F2 generation individual at 6 months of age (see Table 2).

In addition, the amount of collected sperm was greater than 100 μL in all test sections, and was significantly greater than that of the lh hetero F2 generation individual at 6 months of age.

Further, in the lh hetero F2 generation, since the amount of the collected sperm was small, as a result of the insemination of the collected sperm and the eggs collected from the wild type female individual, the fertilization rates were 87.5% and 29.2%, which were less than that of the wild type.

Meanwhile, in the fsh hetero, the fertilization rates of eggs of the wild type individuals were 100% and 95.8%, which were greater than those in the lh hetero, due to the large amount of the collected sperm.

In this manner, the introduction of the exogenous fsh gene made it possible to succeed precocious maturation of the juvenile male individual, that is, to produce gametes at an early stage.

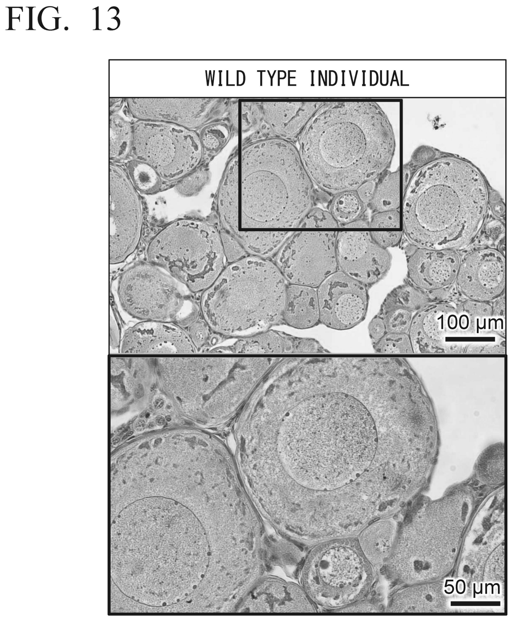

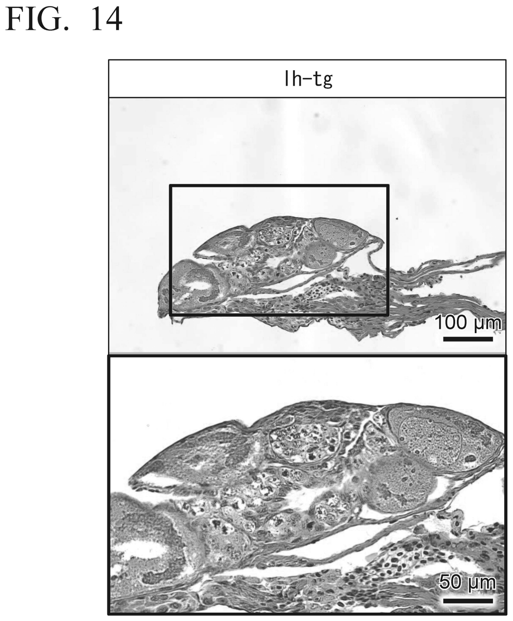

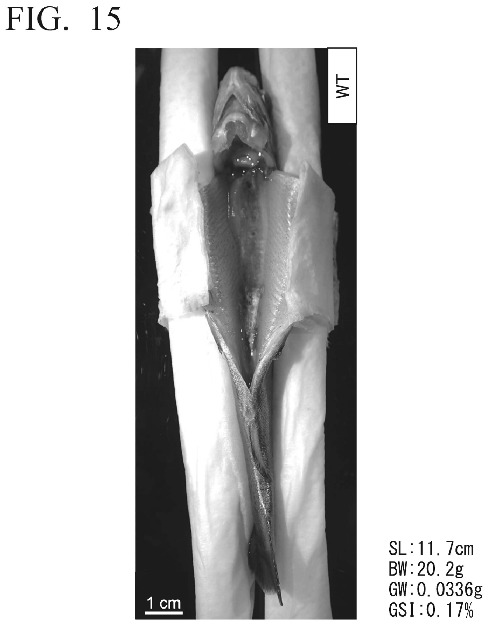

Meanwhile, as shown in FIG. 14 (upper image: HE-stained image of ovary of exogenous lh gene-introduced individual at 12 months of age, scale bar: 100 μm, lower image: scale bar: 50 μm), it was found that in lh hetero female into which the exogenous lh gene derived from hsc:lh had been introduced, the ovaries were not developed at all at 12 months of age, as compared with the wild type female individual shown in FIG. 13 (upper image: HE-stained image of ovary of wild type individual at 12 months of age, scale bar: 100 μm, lower image: scale bar: 50 μm) and FIG. 15 (photograph observing bottom surface of abdomen of wild type individual at 12 months of age in opened state).

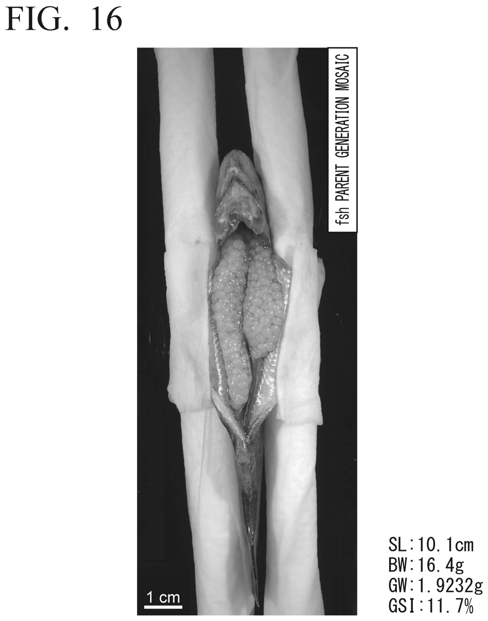

Meanwhile, in the fsh parent generation mosaic female individual into which the exogenous fsh gene derived from hsc:fsh was introduced, as shown in FIG. 16 (photograph observing bottom surface of abdomen of fsh parent generation mosaic individual at 12 months of age in opened state), the clear development of the ovary was confirmed, and the GSI was 11.7%. This is a significantly high value as compared with a GSI of 0.17% of the wild type individual of the same age.

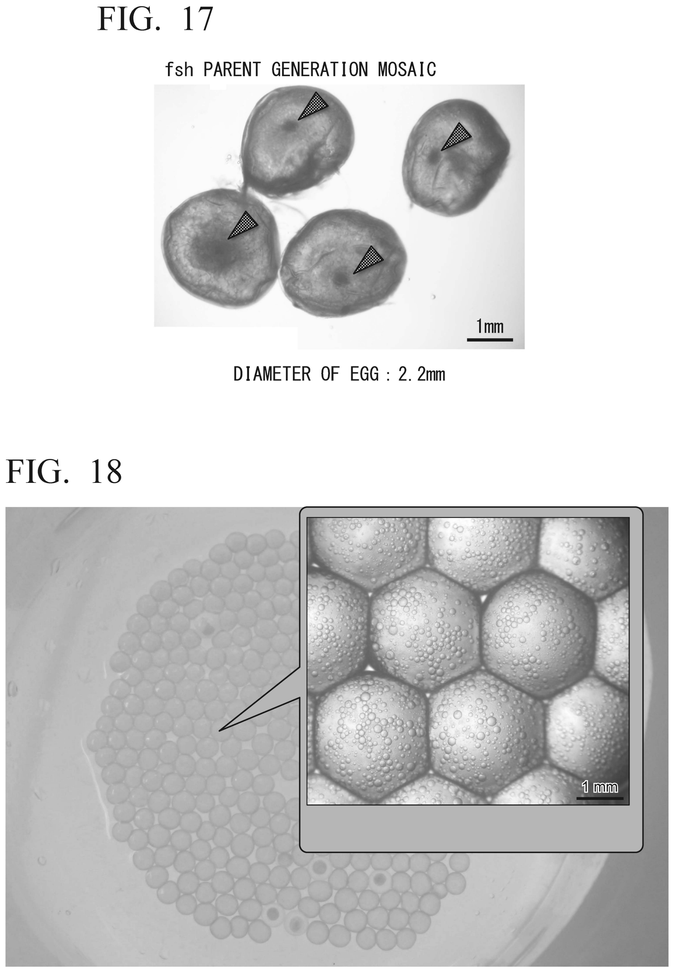

As a result of collecting eggs from the ovary of the fsh parent generation mosaic female individual, immersing the eggs in a Serra's fixative for 5 minutes, and observing the eggs with a stereomicroscope, it was found that a nuclear membrane remained inside each egg as shown in FIG. 17 (stereomicroscope photograph of eggs collected from fsh parent generation mosaic). The nuclear membrane indicates that the eggs are immature.

Thereafter, in a case where the fsh parent generation mosaic female individual was continuously bred, ovulation was confirmed at 14.5 months of age (see FIG. 18). In a case where the eggs were fixed with a Serra's fixative, it was found that the eggs were mature because the nuclear membrane was not confirmed inside the eggs.

As a result of the insemination of sperm of a wild type individual and eggs obtained from an fsh parent generation mosaic individual at 14.5 months of age, the result in which the fertilization rates were 58.3% and 83.3% was obtained as listed in Table 3.

[Table 3]

| Fertilization date | Male parent ♂ | Female parent ♀ | Fertilization | Fertilization rate |
|---|---|---|---|---|
| 2023/2/10 | Wild type | fsh-tg mosaic blue ♀ CO17 | 14/24 | 58.3% |
| | Wild type | Wild type (control) | 23/24 | 95.8% |
| 2023/2/17 | Wild type | fsh-tg mosaic blue ♀ CO16 | 20/24 | 83.3% |
| | Wild type | Wild type (control) | 24/24 | 100.0% |

Therefore, it was clarified that the eggs obtained from the fsh parent generation mosaic individual at 14.5 months of age had a fertilization ability.

Meanwhile, for an individual having a exogenous fsh gene derived from hsc:fsh, the qualification as a surrogate parent (host) was confirmed as follows.

First, sperm collected from an fsh hetero F1 generation male individual (individual obtained by fertilizing sperm collected from an fsh parent generation mosaic individual at 6 months of age and eggs collected from a wild type individual) and eggs collected from a wild type female individual were inseminated and subjected to a triploid treatment to produce an infertile individual that does not produce the individual's own gametes. In addition, the wild type infertile individuals were also produced.

In the triploid treatment, the fertilized egg immediately after fertilization was immersed in running water at 10°C for 10 minutes, quickly moved to a constant-temperature tank at 28°C, and allowed to stand for 15 minutes.

Thereafter, the fertilized eggs were bred in a running water tank at 10.5°C, thereby producing a triploid infertile individual.

Next, each of the 3N fsh hetero and wild type infertile individuals produced as described above, the 2N fsh heterozygous individual and the 2N wild type individual were anesthetized with 2-phenoxyethanol. Thereafter, a cell suspension of spermatogonia (germline stem cells) of vasa-gfp gene-introduced rainbow trout (Yoshizaki et al., 2000; Takeuchi et al., 2002) in which germ cells specifically emit green fluorescence was microinjected into the abdominal cavities of each of the infertile individuals, the 2N fsh heterozygous individuals, and the wild type individuals before and after hatching. This cell suspension was prepared as follows.

First, the vasa-gfp gene-introduced rainbow trout was subjected to an anesthetic treatment with a 2-phenoxyethanol solution (manufactured by FUJIFILM Wako Pure Chemical Corporation), and the abdomen was incised to remove the testis.

Thereafter, the testis was finely cut to prepare testis fragments. The obtained testis fragments were subjected to an enzyme treatment at 20°C for 2 hours with a cell dispersion enzyme solution obtained by mixing 850 μL of L-15/10% FBS, 50 μL of a 0.2% collagenase solution, 50 μL of a 0.17% Dispase II solution, and 50 μL of a DNase-I solution.

During the enzyme treatment, a pipetting treatment was performed every 30 minutes to promote the dissociation of the testis fragments.

After the enzyme treatment, the fragments were subjected to a centrifugal treatment at 10°C and 200 × g for 10 minutes, and washed with H-MEM 5 in which 4.7 g of EAGLE'S MEM, 2.38 g of HEPES, and 460 mL of distilled water were mixed. The operation from the centrifugal treatment to the washing was repeated twice.

Thereafter, the cell aggregates that could not be completely dissociated were removed by filtration through a nylon mesh having a mesh size of 42 μm and a nylon mesh having a mesh size of 20 μm.

This cell solution was collected, the number of dispersed cells was calculated using an improved Neubauer counting chamber (manufactured by HIRSCHMANN), and then the cell concentration was adjusted.

Thereafter, the cell solution was filtered again through a nylon mesh with a mesh size of 20 μm, and a 1 mM Rock inhibitor (Y-27632; Sigma-Aldrich LLC, MO) was added to the cell suspension after filtration such that the final concentration thereof reached 50 μM.

The cell suspension containing spermatogonia prepared as described above was microinjected into the abdominal cavity of each of the above-described infertile individuals, the 2N fsh heterozygous individual, and the wild type individual, and the host fry was transferred to a recovery tank filled with an isotonic solution and bred for one night. Thereafter, the fry was bred in running water at 10.5°C.

20 days after the microinjection, the head of each host fry was removed, the abdomen was opened, and the liver and digestive organs were removed.

Next, using a fluorescence microscope (BX53, manufactured by Olympus Corporation), the presence or absence and the number of green fluorescent cells in the gonads of each host fry were measured, and it was confirmed whether or not the germ cells of the donor vasa-gfp gene-introduced rainbow trout were incorporated into the gonads of the host fry.

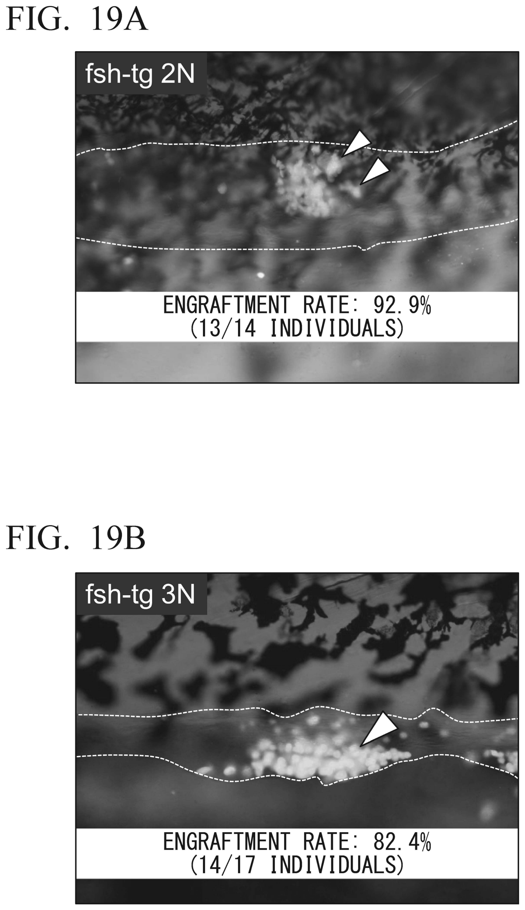

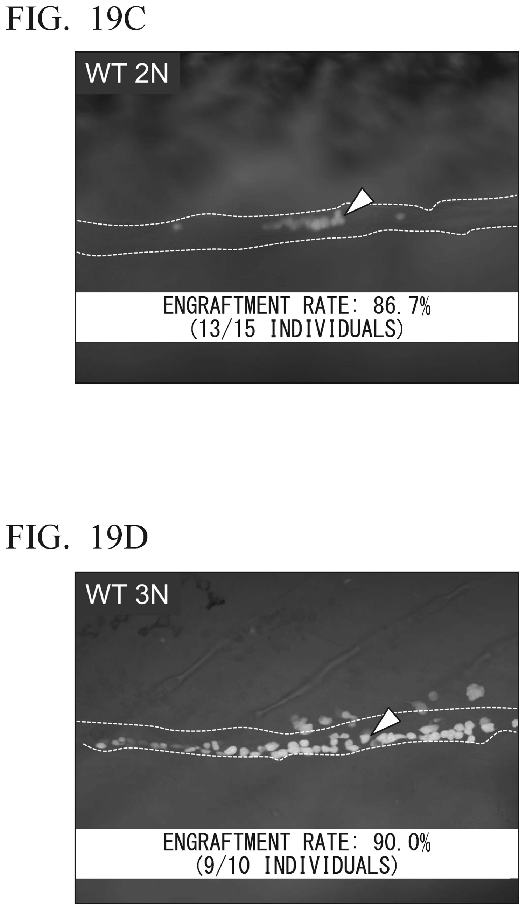

FIG. 19A is a fluorescence image of a gonad of a 2N fsh heterozygous individual, FIG. 19B is a fluorescence image of a gonad of a 3N exogenous fsh-introduced infertile individual, FIG. 19C is a fluorescence image of a gonad of a 2N wild type individual, and FIG. 19D is a fluorescence image of a gonad of a 3N infertile individual having a genome derived from a wild type individual. As shown in the images, in any of the 2N and 3N wild type individuals, and fsh hetero (2N) and 3N individuals into which the exogenous fsh gene was introduced, green fluorescent cells indicated by triangular arrows were engrafted with a high probability in the reproductive ridge indicated by a broken line. This indicates that the spermatogonia microinjected into the abdominal cavity of the host fry moved to the reproductive ridge of the host.

In addition, it was also clarified that in the fsh hetero (2N) and 3N individuals, green fluorescent cells indicated by triangular arrows proliferated more and formed colonies as compared with the 2N and 3N of the wild type individuals.

As shown in the above-described results, it was found that the 2N and 3N individuals having the exogenous fsh gene had the ability to attract and incorporate the transplanted germline stem cells to the reproductive ridge, as in the wild type individuals. Therefore, it is possible to proliferate the introduced germline stem cells and produce gametes by using a surrogate parent.

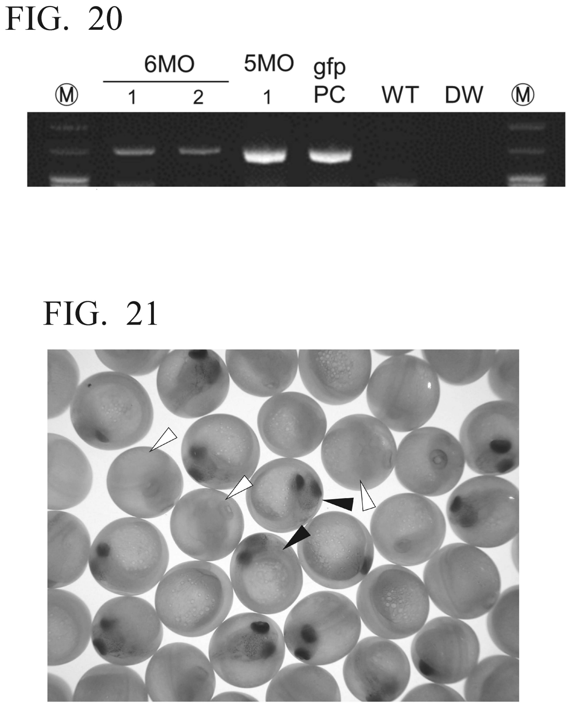

FIG. 20 is an image showing the results of PCR amplification of the gfp gene using the genome of sperm collected from the fsh hetero 3N host at 5 months of age and 6 months of age as a template and electrophoresis.

FIG. 20 shows bands of PCR products amplified from sperm collected from fsh hetero 3N individuals at 5 months of age and 6 months of age after the spermatogonia collected from the gfp gene-introduced rainbow trout are microinjected into the abdominal cavity of the fsh hetero-3N individuals before and after hatching.

In FIG. 20, a PCR product in a case where a genome obtained from a wild type individual is used as a template is injected into a lane of "WT", and a PCR product in a case where distilled water is subjected to PCR is injected into a lane of "DW".

As shown in FIG. 20, a band showing the amplification product of the gfp gene derived from the donor was confirmed from both the sperm collected from the fsh hetero 3N individual at 5 months of age and the sperm collected from the fsh hetero 3N individual at 6 months of age.

FIG. 21 is a microscopic image showing fertilized eggs produced by fertilizing eggs collected from a wild type female individual with sperm collected from an fsh hetero 3N individual which is a host, the sperm being derived from spermatogonia collected from individuals of rainbow trout which are dominant albino gene heterozygotes.

In FIG. 21, among the produced fertilized eggs, the albino individuals are indicated by triangular black arrows, and the albino individuals are indicated by white arrows.

In addition, Table 4 shows the proportion of albino individuals in the next generation produced by fertilizing sperm collected from five fsh hetero 3N individuals (5 months of age) which are hosts, with eggs collected from wild type female individuals.

[Table 4]

|  | 1 | 2 | 3 | 4 | 5 | control |
|---|---|---|---|---|---|---|
| Ejaculate volume (μL) | 11.9 | 8.5 | 9.2 | 9.6 | 55 | - |
| Density of sperm | $2.63 \times 10^8$ | $7.67 \times 10^8$ | $1.4 \times 10^8$ | $1.9 \times 10^9$ | $4.1 \times 10^8$ | - |
| Number of albinos | 22 | 24 | 7 | 79 | 216 | - |
| Number of eyed eggs | 45 | 49 | 12 | 134 | 440 | 331 |
| Number of test eggs | 521 | 331 | 547 | 463 | 551 | 377 |
| Albino rate | 48.9% | 49.0% | 58.3% | 59.0% | 49.1% | - |
| Rate of eyed eggs | 8.6% | 14.8% | 2.2% | 28.9% | 79.9% | 87.8% |

As shown in Table 4, in the next generation obtained by fertilizing the egg collected from the wild type female individual with the sperm collected from the fsh hetero 3N individual, the wild type individual and the albino individual were included in half.

From this, it was suggested that all the sperm produced in the fsh hetero 3N individual as the host was derived from the donor.

Therefore, it was clarified that the fsh hetero 3N individual having the exogenous fsh gene can proliferate the microinjected germline stem cells as a surrogate parent and produce gametes (in other words, has the qualification as a surrogate parent).

In addition, according to the present experiment, it was possible to produce the sperm of the rainbow trout, which usually takes two years to mature, in 5 months and to produce the next generation.

In the present experiment, the same species of germline stem cells were transplanted into the host, but as described below, the same results can be obtained even in a case where the germline stem cells of the Salmonidae are transplanted into the abdominal cavity of the fry of another species of Salmonidae.

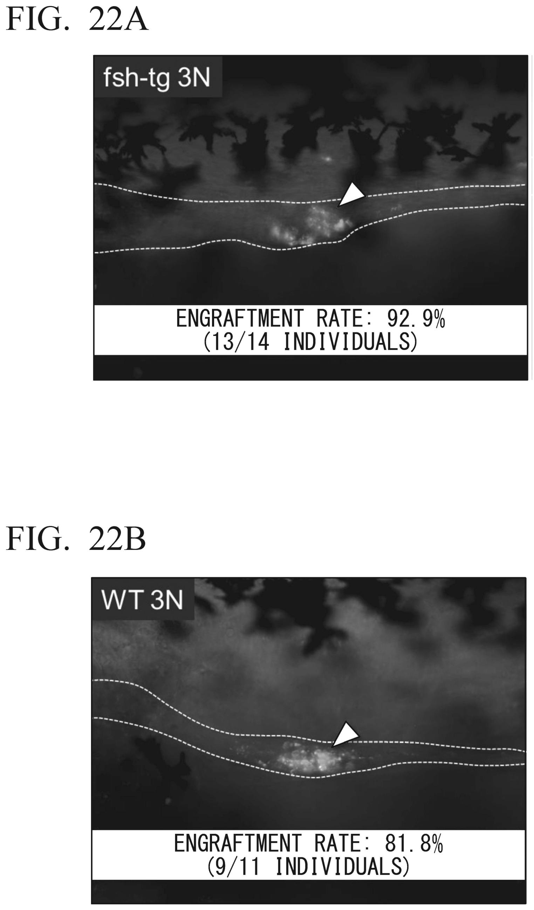

FIG. 22A is a fluorescence image of a gonad of an fsh hetero 3N individual, and FIG. 22B is a fluorescence image of a gonad of a wild type 3N individual.

In the present experiment, the Chinook salmon spermatogonia (germline stem cells) labeled with green fluorescence by the No. 189 antibody (Hayashi et al., 2019; Ichida et al., 2019), which is a specific antibody against the spermatogonia surface antigen, were microinjected into the abdominal cavity of the fsh hetero 3N individuals of the rainbow trout before and after hatching, which are the host fry, and the abdominal cavity of the wild type 3N individuals, and whether or not the Chinook salmon spermatogonia was incorporated into the gonads was confirmed.

As shown in FIGS. 22A and 22B, in both a case where the fsh hetero 3N individual of rainbow trout was used and a case where the wild type 3N individual was used as the host fry, green fluorescent cells indicated by triangular arrows were engrafted with a high probability in the reproductive ridge indicated by a broken line. This result indicates that the spermatogonia derived from the Chinook salmon, which had been microinjected into the abdominal cavity of the host fry, moved into the reproductive ridge of the rainbow trout which was the host.

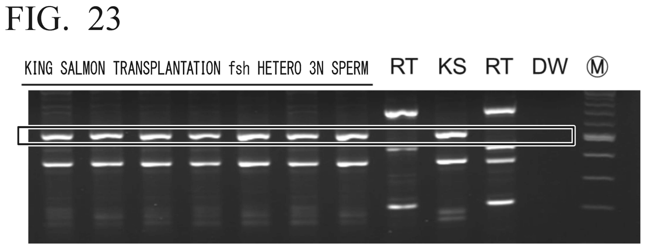

FIG. 23 is an electrophoresis image obtained by performing species discrimination analysis by a restriction fragment length polymorphism (RFLP) method using a genome of sperm collected from fsh hetero 3N individual of rainbow trout at 8 months of age into which spermatogonia derived from Chinook salmon were injected. The vasa gene is PCR amplified as a template, and the amplified fragment is subjected to a restriction enzyme treatment with MboI and subjected to electrophoresis.

Due to the restriction enzyme treatment, the band patterns of the template are different between a case of the sequence derived from the vasa gene of the rainbow trout and a case of the sequence of the Chinook salmon.

In FIG. 23, in the lane of "king salmon transplantation fsh hetero 3N sperm", a PCR product treated with a restriction enzyme MboI, in a case where a genome of sperm collected from an fsh hetero 3N individual of rainbow trout, in which spermatogonia collected from Chinook salmon was microinjected before and after hatching, is used as a template is injected. The fsh hetero 3N individual during the sperm collection was at 8 months of age.

In addition, in the lane of "RT", a PCR product treated with the restriction enzyme MboI in a case where a genome obtained from rainbow trout is used as a template is injected.

In the lane of "KS", a PCR product treated with the restriction enzyme MboI in a case where a genome obtained from Chinook salmon (king salmon) is used as a template is injected. Further, in the lane of "DW", a PCR product treated with the restriction enzyme MboI in a case where distilled water is subjected to PCR is injected.

As shown in FIG. 23, as a result of performing the RFLP analysis using the genome of the sperm collected from the fsh hetero 3N individuals of the rainbow trout that were microinjected before and after hatching of the spermatogonia collected from Chinook salmon, the sperm exhibited the same band pattern as the Chinook salmon.

That is, by injecting the spermatogonia collected from the Chinook salmon into the fsh hetero 3N individual of the rainbow trout before and after hatching, it was possible to produce the sperm derived from the Chinook salmon as a host.

Therefore, in the present experiment, since the sperm of the Chinook salmon was obtained from the fsh hetero 3N individual at 8 months of age, it was successful to produce the sperm of the Chinook salmon, which takes more than 4 years to reach the normal maturity, in just 8 months.

INDUSTRIAL APPLICABILITY

According to the present invention, since the DNA containing a gene that is functionally linked to a constitutive promoter and encodes follicle-stimulating hormone is introduced into fish, it is possible to produce gametes at an early stage from hatching, and thus the present invention can be used industrially.

REFERENCE SIGNS LIST

s1 Vector preparation step
s2 fsh introduction step

**Claims**

1. A method for producing fish, comprising:
   a step of introducing DNA containing a constitutive promoter and a gene functionally linked to the promoter and encoding follicle-stimulating hormone, into a fertilized egg of fish.

2. The method for producing fish according to Claim 1,

   wherein the gene has a base sequence encoding an FSHβ chain and a base sequence encoding a GTHα chain,
   the base sequence encoding the FSHβ chain encodes a protein having an amino acid sequence set forth in SEQ ID NO: 19 or encodes a protein having an amino acid sequence having 30% or more sequence identity with the amino acid sequence set forth in SEQ ID NO: 19, and
   the base sequence encoding the GTHα chain encodes a protein having an amino acid sequence set forth in SEQ ID NO: 20 or encodes a protein having an amino acid sequence having 60% or more sequence identity with the amino acid sequence set forth in SEQ ID NO: 20.

3. The method for producing fish according to Claim 2,
   wherein the promoter is a promoter that controls transcription of an hsc71 gene of fish.

4. The method for producing fish according to Claim 3,
   wherein the DNA contains a 3' untranslated region of the hsc71 gene of fish on a downstream side of the base sequence encoding the GTHα chain.

5. The method for producing fish according to any one of Claims 2 to 4,
   wherein the fish is a female.

6. The method for producing fish according to any one of Claims 2 to 4,
   wherein the fish is a male.

7. The method for producing fish according to any one of Claims 2 to 4,
   wherein the fertilized egg is a fertilized egg of Salmonidae.

8. The method for producing fish according to Claim 7,
   wherein the fertilized egg is a fertilized egg of the genus Oncorhynchus or the genus Salmo.

9. The method for producing fish according to Claim 8,
   wherein the fertilized egg is a fertilized egg of rainbow trout.

10. The method for producing fish according to Claim 8,
wherein the fertilized egg is a fertilized egg of Salmo salar.

11. The method for producing fish according to any one of Claims 2 to 4,
wherein the DNA is linear DNA.

12. Fish,
wherein a constitutive promoter and a gene functionally linked to the promoter and encoding follicle-stimulating hormone are introduced into a genome.

13. The fish according to Claim 12,

wherein the gene has a base sequence encoding an FSHβ chain and a base sequence encoding a GTHα chain, the base sequence encoding the FSHβ chain encodes a protein having an amino acid sequence set forth in SEQ ID NO: 19 or encodes a protein having an amino acid sequence having 30% or more sequence identity with the amino acid sequence set forth in SEQ ID NO: 19, and
the base sequence encoding the GTHα chain encodes a protein having an amino acid sequence set forth in SEQ ID NO: 20 or encodes a protein having an amino acid sequence having 60% or more sequence identity with the amino acid sequence set forth in SEQ ID NO: 20.

14. The fish according to Claim 13,
wherein the promoter is a promoter that controls transcription of an hsc71 gene of fish.

15. The fish according to Claim 14,
wherein a 3' untranslated region of the hsc71 gene of fish is disposed on a downstream side of the base sequence encoding the GTHα chain.

16. The fish according to any one of Claims 13 to 15,
wherein the fish is a female.

17. The fish according to any one of Claims 13 to 15,
wherein the fish is a male.

18. The fish according to any one of Claims 13 to 15,
wherein the fish is Salmonidae.

19. The fish according to Claim 18,
wherein the fish is the genus Oncorhynchus or the genus Salmo.

20. The fish according to Claim 18,
wherein the fish is rainbow trout.

21. The fish according to Claim 18,
wherein the fish is Salmo salar.

22. A method for producing gametes of fish, comprising:
a step of transplanting a germline stem cell of another species of fish into an abdominal cavity of fry of the fish according to any one of Claims 13 to 15.

# FIG. 1

```
        ┌──────────┐
        │  START   │
        └────┬─────┘
             │
             ▼
┌─────────────────────────────┐
│   VECTOR PREPARATION STEP    │──── S1
└──────────────┬──────────────┘
               │
               ▼
┌─────────────────────────────┐
│    fsh INTRODUCTION STEP     │──── S2
└──────────────┬──────────────┘
               │
               ▼
        ┌──────────┐
        │   END    │
        └──────────┘
```

# FIG. 2

| Rainbow trout(Rt) HSC71 promoter | HSC CDS | Rt FSHβ | eCTP | Rt GTHα | Rt HSC 3'UTR |
|---|---|---|---|---|---|
| 2836bp | 12 | 411bp | TOTAL123 | 279bp | 660 |

6×His

pBlueScript II SK(+)

EP 4 692 352 A1

# FIG. 3

SEQ ID NO: 26

HSC71_gene_promoter
2,760    2,780    2,800    2,820    2,840    2,860

TTACAAATGACCAATGTATTTTGATGACCACATTGGCTTCTAGCTGATATTTTTACTGCGTTTGTTGTATTCTTGCATAGTGTCCATTTGTTACCTAACACCATGC

HSC71_gene_promoter    HSC71_5'UTR    Om_HSC_CDC    FSHβ    *PstI*
2,880    2,900    2,920    2,940    2,960

TGTTCTTCATTGTCCAGGTAACATGTCTAAGGGAATGTACTGCACCCACTTAAAGATGCTGCAGCTGGTCGTCATGGCAACGCTGTGGGTGACACCAGTGAGGGCG

FSHβ    *PstI*
2,980    3,000    3,020    3,040    3,060

GGGACAGACTGCAGGTATGGCTGCCGACTAAACAACATGACCATCACCGTGGAGAGAGAGGACTGTCACGGAAGCATCACCGTCACCACCTGCGCCGGCCTGTGCG

FSHβ
3,080    3,100    3,120    3,140    3,160    3,180

AAACAACGGACCTGAACTATCAGAGCACATGGCTGCCGCGCTCCCAGGGGGTGTGTAACTTCAAGGAGTGGTCCTACGAGAAGGTCTACCTGGAAGGCTGTCCATC

FSHβ
3,200    3,220    3,240    3,260    3,280

CGGGGTCAACCCCCTCTTCATACCCGTTGCCAAGAGCTGCGATTGCATCAAATGCAAGACGGACAACACCGACTGTGATCGCATAAGCATGGCAACACCCAGCTGC

FSHβ    eCTP    *BamHI*    Histidine Tag    eCTP
3,300    3,320    3,340    3,360    3,380

ATAGTAAACCCACTAGAAATGTCCTCTTCCTCTAAGGATCCACATCACCATCACCATCACCCATCCCAACCTCTCACATCCACATCCACCCCAACTCCTGGGGCCA

eCTP    GTHa
3,400    3,420    3,440    3,460    3,480

GCAGACGTTCCTCTCATCCCCTCCCAATAAAGACTTCTTATCCAAACAGTGACAAGACAAACATGGGCTGTGAGGAATGCACACTGAAGCCGAACACAATCTTCCC

GTHa
3,500    3,520    3,540    3,560    3,580    3,600

CAACATCATGCAGTGTACAGGCTGTTGCTTCTCCAGAGCTTATCCAACCCCACTACGGTCCAAGCAAACCATGCTGGTTCCCAAGAACATCACCTCTGAAGCCACA

GTHa    *PstI*    Om_HSC_3'UTR
3,620    3,640    3,660    3,680    3,700

TGCTGCGTTGCAAAAGAAGGGGAAAGGGTCACCACCAAGGATGGCTTCCCGGTGACGAACCACACAGAGTGTCACTGCAGCACCTGCTATTACCATAAATCATAAA

Om_HSC_3'UTR
3,720    3,740    3,760    3,780    3,800

CATTCCATTGTTTCTGCAACTACCTCCCCCTAACAAGCAAAGTTTGAAATGTGTTCTACCCTCTTGTGTCTGAGTCTCGTGACTTGTCAAGGAGTGAAATTTGAAT

Om_HSC_3'UTR
3,820    3,840    3,860    3,880    3,900    3,920

AAAGGTTGGGGGATCACATATTTTTCAACTTTGTTTGCTACGCAACAGGGAACAGTAATTTTCTGTTAGCACAACTAAGCTGTCAAATTTCACATGTGTATTTTCT

FIG. 4

FIG. 5

EP 4 692 352 A1

# FIG. 6

DERIVED FROM phsc71P/CDS →    hsc71
| Promotor | CDS |

DERIVED FROM plh/hsc3'UTR →    eCTP
| Lhβ CHAIN | | Gthα CHAIN | hsc71 3' UTR |
6×His

Xho I  EcoR I
| T7 | | | | T3 |
pBlueScript II SK(+)

Xho I, EcoR I
RESTRICTION
ENZYME TREATMENT →

| T7 |                    pBlueScript II SK(+)                    | T3 |

↓ Ligation

hsc71                     eCTP
| T7 | Promotor | CDS | Lhβ CHAIN | | Gthα CHAIN | hsc71 3' UTR | T3 |
6×His

hsc:lh

EP 4 692 352 A1

# FIG. 7

# FIG. 8

# FIG. 9

# FIG. 10A

WILD TYPE INDIVIDUAL AT 6 MONTHS OF AGE

# FIG. 10B

fsh-tg MOSAIC AT 5.5 MONTHS OF AGE

FIG. 11

fsh PARENT GENERATION MOSAIC ♂ AT 13 MONTHS OF AGE

FIG. 12

fsh PARENT GENERATION MOSAIC ♂ AT 13 MONTHS OF AGE

FIG. 13

WILD TYPE INDIVIDUAL

FIG. 14

FIG. 15

SL：11.7cm
BW：20.2g
GW：0.0336g
GSI：0.17%

FIG. 16

SL : 10. 1cm
BW : 16. 4g
GW : 1. 9232g
GSI : 11. 7%

## FIG. 17

fsh PARENT GENERATION MOSAIC

1mm

DIAMETER OF EGG : 2.2mm

## FIG. 18

1 mm

## FIG. 19A

fsh-tg 2N

ENGRAFTMENT RATE: 92.9%
(13/14 INDIVIDUALS)

## FIG. 19B

fsh-tg 3N

ENGRAFTMENT RATE: 82.4%
(14/17 INDIVIDUALS)

FIG. 19C

WT 2N

ENGRAFTMENT RATE: 86.7%
(13/15 INDIVIDUALS)

FIG. 19D

WT 3N

ENGRAFTMENT RATE: 90.0%
(9/10 INDIVIDUALS)

FIG. 20

FIG. 21

## FIG. 22A

fsh-tg 3N

ENGRAFTMENT RATE: 92.9%
(13/14 INDIVIDUALS)

## FIG. 22B

WT 3N

ENGRAFTMENT RATE: 81.8%
(9/11 INDIVIDUALS)

FIG. 23

## INTERNATIONAL SEARCH REPORT

| International application No. |
| --- |
| **PCT/JP2024/012460** |

**A. CLASSIFICATION OF SUBJECT MATTER**

*C12N 15/16*(2006.01)i; *A01K 67/027*(2024.01)i; *C12N 5/075*(2010.01)i; *C12N 5/076*(2010.01)i
FI:   C12N15/16; C12N5/075; C12N5/076; A01K67/027 ZNA

According to International Patent Classification (IPC) or to both national classification and IPC

**B. FIELDS SEARCHED**

Minimum documentation searched (classification system followed by classification symbols)

C12N15/16; A01K67/027; C12N5/075; C12N5/076

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

JSTPlus/JMEDPlus/JST7580 (JDreamIII); CAplus/MEDLINE/EMBASE/BIOSIS (STN)

**C. DOCUMENTS CONSIDERED TO BE RELEVANT**

| Category* | Citation of document, with indication, where appropriate, of the relevant passages | Relevant to claim No. |
| --- | --- | --- |
| Y | YOON, J. Y. et al, Production of bovine nuclear transfer embryos using fibroblasts transfected with single-chain human follicle-stimulating hormone gene, Asian-Australasian Journal of Animal Sciences, 2009, vol. 22, no. 2, pp. 168-173 | 1, 12 |
|  | whole document particularly, abstract, p. 169, fig. 1, p. 169, right column to p. 170 |  |
| A |  | 2-11, 13-22 |
| Y | US 2007/0281883 A1 (ROSENFELD, Hanna) 06 December 2007 (2007-12-06) | 1, 12 |
|  | claims, examples |  |
| A |  | 2-11, 13-22 |
| A | CN 112553205 A (FRESHWATER FISHERIES RESEARCH CENTER OF CHINESE ACADEMY OF FISHERY SCIENCES) 26 March 2021 (2021-03-26) | 1-22 |
|  | claims, examples |  |
| A | US 2018/0064077 A1 (AUBURN UNIVERSITY) 08 March 2018 (2018-03-08) | 1-22 |
|  | claims, examples |  |

☑ Further documents are listed in the continuation of Box C.  ☑ See patent family annex.

| | | | |
| --- | --- | --- | --- |
| \* | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | "&" | document member of the same patent family |
| "P" | document published prior to the international filing date but later than the priority date claimed | | |

| Date of the actual completion of the international search | Date of mailing of the international search report |
| --- | --- |
| **14 June 2024** | **25 June 2024** |

| Name and mailing address of the ISA/JP | Authorized officer |
| --- | --- |
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (July 2022)

# EP 4 692 352 A1

**INTERNATIONAL SEARCH REPORT**

| International application No. |
|---|
| **PCT/JP2024/012460** |

## C. DOCUMENTS CONSIDERED TO BE RELEVANT

| Category* | Citation of document, with indication, where appropriate, of the relevant passages | Relevant to claim No. |
|---|---|---|
| A | 守谷奈津子 ほか, 黄体形成ホルモン遺伝子の未成魚における過剰発現は雄ニジマスを早熟化するか?, 第114回日本繁殖生物学会大会 講演要旨集, 2021, Session ID: P-23, (MORIYA, Natsuko et al.), non-official translation (Does overexpression of luteinizing hormone genes in juveniles cause male rainbow trout to premature?, Proceedings of the 114th Meeting of Society for Reproduction and Development)<br>abstract | 1-22 |
| P, X | 守谷奈津子 ほか, 濾胞刺激ホルモン遺伝子の若齢魚における異所的過剰発現による雌ニジマスの早熟化, 第23回マリンバイオテクノロジー学会大会 講演要旨集, 27 May 2023, p. 67, (MORIYA, Natsuko et al.), non-official translation (Ectopic overexpression of follicle-stimulating hormone gene in juvenile fish leads to precocious maturation of female rainbow trout, Proceedings of the 23rd Annual Meeting of the Japanese Society for Marine Biotechnology)<br>abstract | 1-22 |

Form PCT/ISA/210 (second sheet) (July 2022)

36

**INTERNATIONAL SEARCH REPORT**

| International application No. |
| --- |
| **PCT/JP2024/012460** |

| Box No. I | Nucleotide and/or amino acid sequence(s) (Continuation of item 1.c of the first sheet) |
| --- | --- |

1. With regard to any nucleotide and/or amino acid sequence disclosed in the international application, the international search was carried out on the basis of a sequence listing:

    a. ☑ forming part of the international application as filed.

    b. ☐ furnished subsequent to the international filing date for the purposes of international search (Rule 13*ter*.1(a)),

        ☐ accompanied by a statement to the effect that the sequence listing does not go beyond the disclosure in the international application as filed.

2. ☐ With regard to any nucleotide and/or amino acid sequence disclosed in the international application, this report has been established to the extent that a meaningful search could be carried out without a WIPO Standard ST.26 compliant sequence listing.

3. Additional comments:

Form PCT/ISA/210 (continuation of first sheet) (July 2022)

## INTERNATIONAL SEARCH REPORT
### Information on patent family members

International application No.

**PCT/JP2024/012460**

| Patent document cited in search report | | | Publication date (day/month/year) | Patent family member(s) | | | Publication date (day/month/year) |
|---|---|---|---|---|---|---|---|
| US | 2007/0281883 | A1 | 06 December 2007 | WO | 2007/141778 | A2 | |
| | | | | EP | 2021367 | A2 | |
| CN | 112553205 | A | 26 March 2021 | (Family: none) | | | |
| US | 2018/0064077 | A1 | 08 March 2018 | (Family: none) | | | |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2023050625 A

- JP 2015146184 W

**Non-patent literature cited in the description**

- **MIWA S** ; **YAN L** ; **SWANSON P**. Localization of two gonadotropin receptors in the salmon gonad by in vitro ligand autoradiography. *Biol Reprod*, 1994, vol. 50, 629-642

- **ZAFARULLAH, M.** ; **J. WISNIEWSKI** ; **N. W. SHWORAK** ; **S. SCHIEMAN** ; **S. MISRA** ; **L. GEDAMU**. Molecular Cloning and Characterization of a Constitutively Expressed Heat-Shock-Cognate hsc71 Gene from Rainbow Trout. *European Journal of Biochemistry/FEBS*, 1992, vol. 204 (2), 893-900